# EUROPEAN PATENT SPECIFICATION

(11) **EP 3 119 443 B1**
(45) Date of publication and mention of the grant of the patent: **12.05.2021**
(21) Application number: 15714607.7
(22) Date of filing: 19.03.2015
(51) Int. Cl.: A61L 15/22, A61F 13/53, A61L 15/60

(54) **WOUND DRESSING**
WUNDAUFLAGE
PANSEMENT POUR PLAIE

(30) Priority: 19.03.2014 GB 201404944
(43) Date of publication of application: 25.01.2017
(73) Proprietor: Medtrade Products Limited, Crewe, Cheshire CW1 6GL (GB)
(72) Inventor: HOGGARTH, Andrew, Crewe Cheshire CW2 6TA (GB); WARDE, David, Blackley Manchester M9 7BN (GB); HARDY, Craig, St Dogmaels Cardigan SA43 3BH (GB)
(74) Representative: Wilson Gunn
(86) International application number: PCT/GB2015/050818
(87) International publication number: WO 2015/140565

(56) References cited:
- WO-A1-01/24840
- WO-A1-99/67456
- WO-A2-2010/031995
- GB-A- 2 377 177
- JP-A- 2005 102 790

## Description

The present invention relates to materials for use as or in wound dressings. In particular, the present invention relates to a multilayer material for use as or in a wound dressing and to methods of making the multilayer material.

Topical wound dressings for use in the treatment of wounds or other openings at a physiological target site on a human or animal body which are exuding blood and/or other bodily fluids have been known for some time. The materials used to make the wound dressings act to absorb the blood and/or other bodily fluids, and also stem the flow of them from the body. Materials for wound dressings are described in, for example, WO2010031995 to MedTrade Products Limited, and are commercially available.

WO 99/67456 discloses a fabric comprising first and second webs of gel-forming fibre needled to the first and second sides respectively of a textile fibre scrim.

WO 01/24840 discloses a wound care device comprising chitosan, said chitosan being in the form of fibres having been modified by treatment with acid.

JP 2005 102790 discloses a wound covering material with a chitosan woven or nonwoven fabric and a high water absorbent polymer sheet stacked on the woven or nonwoven fabric.

The management of exudate is of course essential and critical during wound care and surgical procedures. The aim of managing the exudate is essentially to provide a moist wound environment at the wound bed to minimise the risk of maceration, which in turn may reduce the negative impact upon the human or animal body and also shorten the length of time the patient will take to recover.

One material that is reported to have a use in wound dressings is chitosan. Chitosan is a derivative of solid waste from shellfish processing and can be extracted from fungus culture. It is a cationic polymeric material that is insoluble in water. Chitosan is a known haemostat for use in wound dressings. The term 'haemostat' is used herein to refer to any agent which is capable of producing a clot or plug which stops or reduces bleeding when it comes into contact with blood or other bodily fluid, such as wound exudate, from a physiological target site or wound site of a human or animal.

There are many different types of chitosan that may be used as a material in wound dressings, with different absorption properties. The different types of chitosan may have different molecular weights, different degrees of deacetylation, different arrangements of *β*-(1-4)-linked D-glucosamine and N-acetyl-D-glucosamine monomers, different chiral forms or they may be derived from different species or sources (and fungi), or may have been treated differently during manufacture. Each and all of these different variations of chitosan materials are envisaged for use within the present invention.

Chitosan materials can exhibit gelling properties when in the form of a salt. To obtain a chitosan salt, chitosan is typically mixed with an appropriate acid. The gelling properties of chitosan salts make them desirable for use as materials in wound dressings.

However, utilising chitosan salts in a wound dressing typically involves a pretreatment step of reacting chitosan with an appropriate acid to form the salt. The chitosan salt is then incorporated into the manufacturing process for the wound dressing. Also, since a chitosan salt gels upon contact with a fluid, the structural integrity of a topical wound dressing comprising chitosan is also an important consideration.

There therefore remains a need for a material suitable for use as or in a wound dressing that is able to maintain, or provide, a structural integrity to the wound dressing whilst also exposing the wound to a material capable of absorbing the blood and/or other bodily fluids, and also stem the flow of such fluids from the body.

According to the present invention, there is provided a multilayer material comprising first and second layers comprising a chitosan and/or a chitosan derivative and a third layer located between the first and second layers comprising a reinforcing material, wherein the chitosan derivative is selected from the group consisting of partially deacetylated chitin, carboxymethyl chitosan, hydroxyl butyl chitin, N-acyl chitosan, O-acyl chitosan, N-alkyl chitosan, O-alkyl chitosan, N-alkylidene chitosan, O-sulfonyl chitosan, sulfated chitosan, phosphorylated chitosan, nitrated chitosan, alkalichitin, alkalichitosan, or metal chelates with chitosan, wherein the third layer comprises a physiologically acceptable acid selected from the group consisting of formic acid, acetic acid, ascorbic acid, halogen acetic acids, propanoic acid, propenoic acid, lactic acid, succinic acid, acrylic acid, glyoxylic acid, pyruvic acid, hydroxyl propionic/butanoic acid, sulphuric acid, hydrochloric acid, and combinations of any two or more thereof.

Due to the construction of the multilayer material of the present invention, it is not necessary to provide information instructing the user as to which side of the multilayer material is the wound contacting surface, i.e. the surface applied directly to the wound. This has clear advantages in situations where the speed of application of the multilayer material is of upmost importance, such as in cases of severe bleeding, since a user such as an emergency responder can apply the multilayer material directly to the wound site without consideration of its orientation.

The term 'wound' is used herein to refer to any breach or opening in the skin or subcutaneous tissue at a physiological target site of a human or animal. Typically, the present invention relates to a physiological target site of a human. The term physiological target site may also be referred to herein as a wound site.

The term 'wound dressing' is used herein to refer to materials placed on a wound at a wound site that have absorbent, gelling, adhesive or protective properties. The wound dressings are not limited to a particular size or shape. The wound dressings may be placed in direct or indirect contact with the wound.

There are described herein multilayer materials wherein:
the physiologically acceptable acid is located in either the first or second layer of the multilayer material;
the physiologically acceptable acid is located in the first and second layers of the multilayer material. In such a material, the third layer may contain no acid or may be substantially free of acid;
the physiologically acceptable acid is located in the third layer of the multilayer material. In such a material, the first and second layers may contain no acid or may be substantially free of acid;
the first and second layers both comprise chitosan and a physiologically acceptable acid and the third layer is substantially free of acid;
the first and second layers both comprise chitosan and either the first or second layer further comprises a physiologically acceptable acid and the third layer is substantially free of acid;
the first and second layers both comprise a chitosan derivative and a physiologically acceptable acid; and the third layer is substantially free of acid;
the first and second layers both comprise a chitosan derivative and either the first or second layer further comprises a physiologically acceptable acid; and the third layer is substantially free of acid;
one of the first or second layers comprises chitosan and the other comprises a chitosan derivative and both first and second layers further comprise a physiologically acceptable acid; and the third layer is substantially free of acid;
one of the first or second layers comprises chitosan and the other comprises a chitosan derivative and either the first or second layers further comprise a physiologically acceptable acid; and the third layer is substantially free of acid;
the first and/or second layers comprise a mixture of chitosan and a chitosan derivative and a physiologically acceptable acid; and the third layer is substantially free of acid;
the first and/or second layers comprise a mixture of chitosan and a chitosan derivative and either the first or second layers further comprise a physiologically acceptable acid; and the third layer is substantially free of acid;

The following embodiments of the multilayer material of the present invention are envisaged:
the third layer comprises a reinforcing material and a physiologically acceptable acid; and the first and second layers are substantially free of acid;
the third layer comprises a reinforcing material and a physiologically acceptable acid; and the first or second layer also comprises an acid;
the first, second and third layers all comprise a physiologically acceptable acid.

Typically, the first and second layers comprise chitosan with a physiologically acceptable acid.

Typically, the first and second layers have substantially the same composition.

The first and/or second layers may be woven or non-woven. Preferably, the first and/or second layers are non-woven.

The first and/or second layers may be absorbable or non-absorbable. As such, the first and/or second layers may consist of or comprise an absorbent material or a non-absorbent material.

The first and/or second layers may further comprise a carrier material for the acid. In some embodiments, the carrier material does not substantially gel when exposed to a fluid but does gel when brought together with the chitosan and/or chitosan derivative and exposed to a fluid.

The carrier material may have the physiologically acceptable acid associated therewith. The carrier material can act as a carrier for the physiologically acceptable acid. In such embodiments, the acid should not be bonded to the carrier material.

The carrier material may comprise a non-gelling material that can absorb or act as a carrier for a physiologically acceptable acid. Typically, the acid is absorbed in, or coated onto, the carrier material. Typical materials include, but are not limited to polymers such as cellulose, cellulose derivatives (e.g. ethyl cellulose, methyl cellulose, etc), cotton, alginate, viscose, polypropylene, polyethylene or any combination of such materials.

The chitosan and/or chitosan derivative may be mixed with the carrier material. Alternatively, the chitosan and/or chitosan derivative may be segregated in separate layers or sections of the first and/or second layers.

Typically, the carrier material is fibrous. The chitosan and/or chitosan derivative and the carrier material may be combined together to make a non-woven fabric, and are typically carded or needled together.

The reinforcing material provides a means of maintaining the structural integrity of the multilayer material. As will become apparent, it may also have additional properties and advantages that make it particularly beneficial as part of the multilayer material of the present invention.

For example, the structural integrity provided by the reinforcing material enables the multilayer material of the present invention to be removed from a wound in one piece. In cases where wound dressings tear or rip when in use, care is required to ensure that all of the dressing is removed from the wound so as to decrease the risk of foreign body responses within the wound, potentially leading to further wound breakdown.

Where a dressing has a low tensile strength, typically observed for gelling dressings, the risk of tearing during use and upon removal increases. Further, for wounds such as burns, venous leg ulcers and pressure sores in higher exuding conditions, gelling dressings can lose tensile strength and tear on removal. For wounds moving from an exuding state to a dry state, adherence to the wound can also cause tearing on removal. When a wound dressing is used for a trauma wound, i.e. as a haemostat, during application, post absorbance of blood and fluids, the gelling dressings can tear and, likewise, on removal in deeper wounds, they can have a tendency to tear, resulting in the surgical team needing to investigate and ensure all dressing is removed. It is therefore important that the tensile strength of gelling dressings does not affect the performance of the dressing in terms of fluid handling but is high enough to enable removal of the dressing in one piece.

The first and/or second layers of the multilayer material may have a dry tensile strength of greater than 5N/25mm, sometimes greater than 1 0N/25mm or greater than 20N/25mm. When wet, the tensile strength of the first and/or second layers will generally reduce. The first and/or second layers of the multilayer material may have a wet tensile strength of less than 10N/25mm, sometimes less than 5N/25mm or less than 2N/25mm. Where the tensile strength is reduced, the dressing can be more prone to tearing or ripping. Beneficially, the intermediate layer of reinforcing material prevents the multilayer material of the present invention from tearing on removal from a wound.

The tensile strength necessary to prevent tearing or ripping of the multilayer material upon removal from the wound can vary depending on the nature of the wound, the volume of exudate, etc.

The multilayer material may have a dry tensile strength of greater than 10N/25mm; greater than 20N/25mm; greater than 30N/25mm; or greater than 50N/25mm.

The multilayer material may have a wet tensile strength of greater than 5N/25mm; greater than 10N/25mm; greater than 16N/25mm; or greater than 20N/25mm.

The reinforcing material may consist of, or may comprise, a non-absorbent material. The non-absorbent material may be selected from viscose, polyethylene, nylon, acrylics, semi-synthetics, cellulose, olefins, or combinations thereof. Preferably, the polyethylene is high-density polyethylene.

The first, second and third layers may consist of, or may comprise, a non-absorbent material and may be non-absorbable. Alternatively, the first, second and third layers may consist of, or may comprise, absorbent material and may be absorbable. Alternatively still, one of the first, second and third layers may consist of, or may comprise, a non-absorbent and the other two may consist of, or may comprise, an absorbent material, and vice versa.

In some embodiments, the first and second layers may consist of, or may comprise, an absorbent material and the third layer may consist or, or may comprise, a non-absorbent material. For example, the first and second layers may be absorbent layers and the third layer may be non-absorbable.

In some embodiments, the first and second layers may consist of, or may comprise, a non-absorbent material and the third layer may consist of, or may comprise and absorbent material. For example, the first and second layers may be non-absorbent and the third layer may be absorbent.

The reinforcing material may comprise textile fibres.

The reinforcing material may consist of, or may comprise, an absorbent material.

The term 'absorbent material' is used herein to refer to a physiologically acceptable material that is capable of absorbing fluid, such as wound exudate.

In one embodiment, the reinforcing material may comprise a mixture of non-absorbent and absorbent materials. For example, the reinforcing material may comprise viscose together with an absorbent material.

In one embodiment, the reinforcing material may consist essentially of an absorbent material.

The absorbent material referred to herein may be a superabsorbent material.

The term 'superabsorbent material' is used herein to refer to a hydrophilic material that is water-swellable, but not water soluble, and which is capable of absorbing fluid to greater than 2000% with a fluid retention of greater than 85%. Preferably, the superabsorbent material is capable of absorbing fluid to greater than 2500% with a fluid retention of greater than 90%.

The term 'water-swellable' is used herein to refer to a material that, when contacted with water or water-containing fluid, will absorb the fluid and swell, but will not substantially dissolve in that fluid.

The term 'water soluble' is used herein to refer to a material that, when contacted with water or a water-containing fluid, will dissolve in that fluid.

The superabsorbent material may be selected from polymeric materials such as poly(vinyl) alcohol (PVA), poly(ethylene oxide) (PEO) and poly(acrylic acid).

The superabsorbent material may be chemically modified. For example, the superabsorbent material may be a polymeric material obtained by graft polymerisation of acrylic acid onto a chain of carboxymethyl cellulose.

The superabsorbent material may comprise a chemically modified material selected from starch, cellulose and polymeric materials such as poly(vinyl alcohol) (PVA), poly(ethylene oxide) (PEO), and poly(acrylic acid). The poly(acrylic acid) may be a partially neutralised, lightly cross-linked poly(acrylic acid).

The terms "cross-linking" and "cross-linked" are used herein to refer to two or more polymer chains being linked by a primary bond, such as a covalent bond.

The term "lightly cross-linked" is used herein to refer to embodiments wherein the number of cross-linking primary bonds in the superabsorbent material is less than the total number of possible cross-linking bonds.

In some embodiments, the superabsorbent material is selected from polymeric materials such as PVA, PEO, and poly(acrylic acid), preferably a partially neutralised, lightly cross-linked poly(acrylic acid).

Typically, the superabsorbent material is a partially neutralised, lightly cross-linked poly(acrylic acid).

The reinforcing material may comprise a blend of a superabsorbent material with a non-absorbent material. The ratio of superabsorbent material to non-absorbent material in the blend may be from 50:50 to 70:30. In some embodiments, the reinforcing material comprises a 50:50 blend of polyacrylate fibres and viscose fibres. In further embodiments, the reinforcing material comprises a 70:30 blend of polyacrylate fibres and viscose fibres.

Typically, the third layer has an absorbance capacity of greater than 2000%. Preferably, the absorbance capacity is greater than 2500%.

Typically, the third layer has a fluid retention of greater than 85%. Preferably, the fluid retention is greater than 90%.

In one embodiment, the third layer is capable of absorbing fluid to greater than 2000% with a fluid retention of greater than 85%. In one embodiment, the third layer is capable of absorbing fluid to greater than 2500% with a fluid retention of greater than 90%.

The non-absorbent, absorbent and/or superabsorbent materials referred to above may be in the form of fibres. Typically, the non-absorbent, absorbent and/or superabsorbent materials are in the form of fibres. The length of the fibres can be up to 100mm, and is typically from 20-75mm, more typically from 32 to 51mm.

The reinforcing material may be in the form of a woven or non-woven fibrous layer.

The chitosan and/or chitosan derivative may be in any available form, such as for example, fibres, granules, powder, flakes, sheet, foam, freeze dried foam, compressed foam, film, perforated film, beads, and combinations of two or more of the aforesaid. Typically, the chitosan and/or chitosan derivative is in the form of fibres. The fibres can be of any desired diameter or length and can be formed into a textile fabric or a pad for use. The fibres may be woven or non-woven. Preferably, the fibres are non-woven.

Typically, the molecular weight of the chitosan used in the multilayer material of the present invention is less than about 2,000,000, more typically less than about 1,000,000, and even more typically less than about 500,000, and most typically less than about 175,000.

The viscosity of the chitosan may typically be less than about 1000 cps, more typically less than about 500 cps, even more typically less than about 300 cps. Advantageously, the viscosity is from about 40 to about 200 cps when measured on a Brookfield viscometer at 20°C.

The term 'chitosan derivative' is used herein to refer to a partially deacetylated chitin, which may have different percentages of deacetylation, as desired. Typically, the partially deacetylated chitin suitable for use in the present invention has a deacetylation degree above about 50%, more typically above about 75% and most typically above about 85%. Also included within the term 'chitosan derivative' are reaction products of chitosan with other compounds. Such reaction products are carboxymethyl chitosan, hydroxyl butyl chitin, N-acyl chitosan, O-acyl chitosan, N-alkyl chitosan, O-alkyl chitosan, N-alkylidene chitosan, O-sulfonyl chitosan, sulphated chitosan, phosphorylated chitosan, nitrated chitosan, alkalichitin, alkalichitosan, or metal chelates with chitosan.

In embodiments comprising a chitosan derivative in the first and/or second layers, the chitosan derivative is preferably a partially deacetylated chitin.

The physiologically acceptable acid is an organic acid and/or an inorganic acid, including carboxylic acids and monovalent, divalent or multivalent acids, selected from the group consisting of formic acid, acetic acid, ascorbic acid, halogen acetic acids, propanoic acid, propenoic acid, lactic acid, succinic acid, acrylic acid, glyoxylic acid, pyruvic acid, hydroxyl propionic/butanoic acid, sulphuric acid, hydrochloric acid, and combinations of any two or more thereof.

Typically, the organic acid is selected from carboxylic acids such as formic acid, acetic acid, ascorbic acid, halogen acetic acids (such as fluoro- or chloroacetic acid), propanoic acid, propenoic acid, lactic acid, succinic acid, acrylic acid, glyoxylic acid, pyruvic acid, hydroxyl propionic/butanoic acid and combinations of any two or more thereof.

Typically, the physiologically acceptable acid is selected from carboxylic acids such as lactic acid, acetic acid, succinic acid and combinations of any two or more thereof.

In one embodiment, the organic acid is lactic acid.

Typically, the inorganic acid is selected from hydrochloric acid and sulphuric acid, or a combination thereof.

The physiologically acceptable acid may be an acid that is already present in the human or animal body. This is advantageous in facilitating the bioacceptability of the multilayer material of the present invention, or wound dressing comprising the multilayer material, as it mixes with wound exudates at the wound site.

The physiologically acceptable acid may be coated onto the chitosan, chitosan derivative and/or reinforcing material as desired or as appropriate. For example, the chitosan, chitosan derivative and/or reinforcement material may be in the form of fibres, granules, flakes or a powder, wherein the fibres, granules, flakes or powder particles are coated with a physiologically acceptable acid, such as for example lactic acid.

The first and/or second layers may further comprise a carrier material which can absorb, or be coated with, the physiologically acceptable acid.

Also described herein is a multilayer material wherein the first and second layers both comprise chitosan fibres and lactic acid; and the third layer comprises poly(acrylic acid) fibres. Preferably, the chitosan fibres are coated with lactic acid.

Similalry described herein is a multilayer material with first and second layers comprising chitosan fibres, wherein the first and/or second layer further comprises a carrier material having lactic acid coated thereon; and a third layer comprising poly(acrylic acid) fibres. The chitosan fibres may be mixed with the carrier material or may be segregated in separate layers or sections to make up the first and/or second layers. The carrier material is preferably in the form or fibres.

As referred to herein, the materials used in the multilayer material of the present invention, e.g. chitosan, chitosan derivative, reinforcing material and carrier material, may all be in fibrous form. The fibres can typically have a minimum average length of about 3mm and a maximum length of about 500mm, more typically no more than about 76mm. The typically preferred length of fibres is at least 10mm, more preferred at least 38mm and most preferred at least 51mm.

Alternatively, the materials used in the multilayer material of the present invention may comprise nano-fibres. The term 'nano-fibres' is used herein to refer to fibres having a diameter of no more than about 100 microns. Similarly, the length of the nano-fibres is typically no more than about 100 microns.

The multilayer material has been described herein as comprising first, second and third layers, although it may comprise further layers, such as fourth, fifth, sixth, seventh, eighth, ninth, tenth layers, or more.

The further layers may comprise any of the features referred to herein in relation to the first, second or third layers.

The further layers may additionally or alternatively comprise components of a wound dressing, such as for example, backing, adhesive and/or wound contact materials. Preferably, however, the two outermost layers of the multilayer material comprise the first and second layers as described herein. This allows the multilayer material, once removed from any packaging, to be applied directly to a wound without consideration as to its orientation or preparation.

The backing may comprise medical grade sheet materials such as but not limited to polymer films, thin foams and fabrics e.g polyurethane films, polyurethane foams, nonwoven fabrics, etc.

Suitable skin contact adhesives may include, but are not limited to, acrylate, silicone, or polyurethane based adhesives. They can be based on hydrogels and can be porous to moisture with a high moisture vapour transmission rate. They can be applied from water emulsions, solvents or using hot melt systems. The adhesives should have a good skin tack but give minimal skin trauma on removal. They can constitute 100% coverage of the backing, or a partial coverage thereof in the form of a pattern or mesh.

Suitable wound contact materials may include, but are not limited to, non-adherent layers which give very low or no adhesion to skin, wicking layers to speed up the absorption of fluid, active carrier layers for delivery of a therapeutic material (such as a pharmaceutical, haemostat, antimicrobial, wound healing agent, or scar reducing agent) and adhesive layers to help in holding the dressing in place while potentially reducing trauma on removal. They can be based on a polymer mesh, a fabric (e.g. nonwoven), and a hydrogel adhesive or partial adhesive coverings.

According to a further aspect of the present invention, there is provided a wound dressing comprising a multilayer material as defined herein.

The wound dressing comprising the multilayer material may be in fibrous form, such as in the form of a nonwoven which is structurally capable of being applied to the wound and removed in one piece.

The multilayer material of the present invention, or a wound dressing comprising such multilayer material, may also comprise additional components in the first, second, third or further layer. Such additional components include, but are not limited to, pharmaceutical agents; wetting agents such as surfactants; growth factors; cytokines; agents which absorb agents which delay healing such as MMP's (matrix metalloproteinases) and elastase; and/or another wound dressing component, such as calcium, vitamin K, fibrinogen, thrombin, factor VII, factor VIII, clays such as kaolin, oxidised regenerated cellulose, gelatin, or collagen, etc.

Typical levels of any of these additional components could be from about 50 ppm up to about 50% by weight of the multilayer material. More typical levels would be less than 10%, still more typically less than about 5% by weight of the multilayer material. Additional components comprising less than about 1% by weight of the multilayer material is also envisaged by the present invention.

Typically, the first and second layers of the multilayer material of the present invention are suitable for wound contact. Thus, the multilayer material of the present invention, or a wound dressing comprising the multilayer material, can be applied to a wound such that the first or second layer as described herein comes into direct contact with the wound and wound exudate. This allows the chitosan and/or chitosan derivative and, typically, the physiologically acceptable acid, to come into contact with wound exudates as soon as possible.

When used in wound care applications, chitosan is first converted into a water soluble salt which can gel on contact with fluid, such as wound exudate.

The solubility of the chitosan salt is dependent on the volume of physiologically acceptable acid. If a greater volume of acid is present, the chitosan fibres swell and breakdown more easily in fluid, whereas with a lesser volume of acid the fibres do not swell as much and breakdown less readily. These effects are dependent on the nature of the acid and the chitosan salt.

For optimum utility as a wound dressing it is preferable for the chitosan fibres to swell when contacted with fluid, but not breakdown for up to seven days. For example, the chitosan salt is soluble in blood to form a gel which helps stem blood flow. Thus, it is advantageous to have the chitosan and the physiologically acceptable acid come into contact with wound exudates as soon as possible so as to expedite the formation of the salt.

Chitosan salts are ideally suited for the applications described herein as chitosan is readily broken down in the body. Chitosan is converted to glucosamine by the enzyme lysozyme and is therefore excreted from the body naturally. It is not necessary to remove chitosan from the body. Furthermore, chitosan salts exhibit mild antibacterial properties and as such their use reduces the risk of infection.

It is common in existing wound dressings comprising chitosan to first convert the chitosan into a chitosan salt prior to its incorporation into the wound dressing. This practice has disadvantages in that it is costly and time consuming. In the multilayer material of the present invention, the desired chitosan salt is formed *in-situ* once the multilayer material has been applied to the wound and at least partially mixed with the wound exudates. Thus, the multilayer material of the present invention preferably does not contain a chitosan salt prior to its use, e.g. its contact with a fluid, such as wound exudate.

As referred to herein, the multilayer material of the present invention may comprise a chitosan, chitosan derivative, reinforcement material and/or carrier material coated with a physiologically acceptable acid. It is beneficial to coat the materials with the acid prior to inclusion in the multilayer material as this removes the requirement to pre-treat the chitosan and/or chitosan derivative with acid to form the corresponding salt. Instead, the chitosan and/or chitosan derivative may form the corresponding salt *in situ* upon reaction with the physiologically acceptable acid when the multilayer material contacts bodily fluid, such as wound exudate. Whilst not wishing to be bound by theory, it is thought that the wound exudates dissolve the acid, thus allowing it to mix with the chitosan and thereby facilitate the creation of the salt.

The chitosan salt or chitosan derivative salt obtained is determined by the physiologically acceptable acid or acids incorporated into the multilayer material of the present invention. For example, if the acid is lactic acid, the salt formed in situ will be the lactate salt of chitosan or the chitosan derivative; if the acid is acetic acid, the salt formed in situ will be the acetate salt of chitosan or the chitosan derivative, etc.

Where chitosan is used in the multilayer material of the present invention, suitable chitosan salts that could be formed *in situ* during use of the multilayer material include, but are not limited to, chitosan acetate, chitosan lactate, chitosan succinate, chitosan malate, chitosan acrylate, chitosan formate, chitosan ascorbate, chitosan fluoroacetate, chitosan chloroacetate, chitosan propanoate, chitosan glyoxylate, chitosan pyruvate, chitosan sulphate or chitosan chloride. More typically, the chitosan salt formed in situ during use of the present invention is chitosan lactate.

It has also been discovered that the physiologically acceptable acid can be coated onto a material in any layer of the multilayer material. Preferably, it is coated onto the chitosan and/or chitosan derivative.

According to a further aspect of the present invention, there is provided a method of manufacturing a multilayer material comprising the steps of:
(a) providing first and second layers comprising chitosan and/or a chitosan derivative;
(b) attaching the first and second layers to a third layer comprising a reinforcing material, such that the third layer is located between the first and second layers,
wherein the third layer further comprises a physiologically acceptable acid selected from the group consisting of formic acid, acetic acid, ascorbic acid, halogen acetic acids, propanoic acid, propenoic acid, lactic acid, succinic acid, acrylic acid, glyoxylic acid, pyruvic acid, hydroxyl propionic/butanoic acid, sulphuric acid, hydrochloric acid, and combinations of any two or more thereof.The method of manufacturing a multilayer material as described herein may comprise the steps of:
(a)
   (i) mixing a chitosan and/or chitosan derivative with a non-aqueous solvent; and
   (ii) mixing a reinforcing material or carrier material with a solvent;
(b) adding a physiologically acceptable acid selected from the group consisting of formic acid, acetic acid, ascorbic acid, halogen acetic acids, propanoic acid, propenoic acid, lactic acid, succinic acid, acrylic acid, glyoxylic acid, pyruvic acid,
   hydroxyl propionic/butanoic acid, sulphuric acid, hydrochloric acid, and combinations of any two or more thereof to the or each mixture;
(c) removing the solvent from the or each mixture to provide an acid coated component(s) being an acid coated chitosan, an acid coated chitosan derivative, a mixture of acid coated chitosan and chitosan derivative, an acid coated reinforcing material and/or an acid coated carrier material;
(d) attaching first and second layers comprising chitosan and/or a chitosan derivative to a third layer located between the first and second layers comprising a
   reinforcing material, wherein one or more of the first and second layers comprises the acid coated chitosan, chitosan derivative and/or acid coated carrier material; and wherein the third layer comprises the acid coated reinforcing material.

The acid coated component(s) may be partially or completely coated with acid.

The acid coated component(s) may be in a physical form suitable for forming into layers. The acid coated component(s) may be in the form of coated fibres, granules, flakes, powder particles or the like. The fibres, granules, flakes, powder particles or the like may be completely or partially coated with acid.

In practice, the acid coated component(s) may comprise a mixture of fibres, granules, flakes, powder particles or the like, some of which are partially coated and some of which are completely coated with acid.

In some embodiments, one or more of the first and second layers consists of, or consists essentially of, an acid coated chitosan, an acid coated chitosan derivative and/or acid coated carrier material with chitosan or a chitosan derivative; and the third layer consists of, or consists essentially of, an acid coated reinforcing material.

Typically, the acid coated component(s) comprises at least partially coated chitosan or a chitosan derivative, preferably at least partially coated chitosan. In some embodiments, the chitosan or chitosan derivative are fully coated with acid.

Typically, the first and second layers are the same.

In some embodiments, one or more of the first and second layers consists of, or consists essentially of, the acid coated chitosan and/or chitosan derivative.

The physiologically acceptable acid may alternatively be coated onto the chitosan, chitosan derivative, carrier material and/or reinforcing material using, for example, a dip batch or spray system, or any other suitable coating technique known to the skilled person.

The chitosan, chitosan derivative, carrier material and/or reinforcing material may be provided in a sterile or non-sterile form. Where the material is initially provided in a sterile form, sterilisation may be carried out using any of the methods conventionally known in the art, such as gamma irradiation, electron beam treatment, heat treatment, x-ray, etc., or it may alternatively be carried out by treatment using ethylene oxide. Sterilisation using ethylene oxide is preferred. A material in a non-sterile form may be provided in combination with one or more preservatives. However, it is preferred that the multilayer material is provided in a pre-sterilised form.

In one embodiment, the chitosan raw material may first be washed to reduce the presence of endotoxins prior to its incorporation into the multilayer material of the present invention. The washing step may be carried out by contacting the chitosan and/or chitosan derivative with an alkali solution to form a mixture, and then leaving the mixture for a period of time, which may be as short as about one minute or less to as long as 12 hours or more, before finally drying the mixture. In some embodiments, the mixture is left for a period of 7 days or more; or 14 days or more. The term 'alkali solution' is used herein to refer to a solution having a pH value of greater than pH 7.5.

The concentration of alkali solution used in the process may be from about 0.01M to about 1M. Typically, the concentration of alkali solution is from about 0.02M to about 0.2M, more typically 0.1M.

The quantity of alkali solution to chitosan may be in the range of from about 1 part chitosan and/or chitosan derivative to about 10 parts alkali solution up to about 10 parts chitosan to about 1 part alkali solution. Typically, the quantity of alkali solution to chitosan is about 1 part alkali solution to about 2 parts chitosan, more typically about 1 part alkali solution to about 1 part chitosan.

The alkali solution may comprise an alkali or alkaline earth component selected from the following, either alone or in combination: metal hydroxides, metal carbonates, metal bisulphites, metal persilicates, conjugate bases and ammonium hydroxide. Suitable metals include sodium, potassium, calcium, or magnesium. Typically, the alkali component is sodium hydroxide, potassium hydroxide or sodium carbonate. Typically, sodium hydroxide is used.

The reinforcing material and/or the multilayer material of the present invention may be washed to reduce the presence of endotoxins using the method described above.

The multilayer material of the present invention, or wound dressing comprising such multilayer material, is typically sterilised prior to packaging using any of the methods described herein. This enables the physician or emergency responder to use the multilayer material or wound dressing directly from the packaging, thus saving further time.

As alluded to hereinbefore, when using the multilayer material of the present invention, or a wound dressing comprising such a multilayer material, a chitosan salt is prepared *in situ* when the physiologically acceptable acid dissolves in the wound exudate and contacts the chitosan. The dissolved acid reacts with the chitosan and/or chitosan derivative to form the corresponding salt.

It has been discovered that, in order to avoid a chitosan salt forming during the process of coating the chitosan and/or chitosan derivative with acid, water cannot be present, as it has been observed that water can cause the material to gel during production. As such, a non-aqueous solvent is used when preparing an acid coated chitosan or chitosan derivative.

The non-aqueous solvent may be selected from isopropyl alcohol, dichloromethane, tetrahydrofuran, ethanol, or combinations thereof.

The acid coated chitosan or chitosan derivative layers may be attached to the reinforcing material by heat bonding or needle punching.

Where the acid coated chitosan and/or chitosan derivative layers are attached to the reinforcing material using heat bonding, this may comprise, or consist of, the use of a physiologically acceptable adhesive.

The adhesive material may comprise, or consist of, a pressure-sensitive adhesive, a heat-bonding adhesive, or the like. Typically, the adhesive material is a pressure sensitive adhesive.

The adhesive may be polymeric. Typically, the adhesive material is selected from acrylic adhesives, polyester adhesives, polyurethane adhesives, and silicone adhesives. In one embodiment, the adhesive is polycalprolactone.

In some embodiments, the acid coated chitosan or chitosan derivative may be attached to the reinforcing material by locating a pressure sensitive adhesive there between and applying pressure to bring the acid coated chitosan or chitosan derivative and the reinforcing material together.

According to a further aspect of the present invention, there is provided a multilayer material as defined herein, or a wound dressing as defined herein, for use in the treatment of a wound, in absorbing fluid discharged from a physiological target, or in stemming a flow of a fluid discharged from a physiological target site.

Embodiments of the present invention will now be further described with reference to the following non-limiting examples and accompanying figure in which:

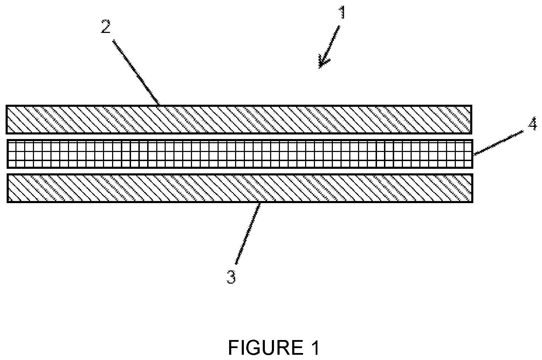

Figure 1: is a representation of a multilayer material of the present invention.

Referring to Figure 1, there is shown a multilayer material (1) comprising first and second layers (2, 3) and a third layer (4). The first and second layers (2, 3) comprise chitosan and/or a chitosan derivative and the third layer (4) comprises a reinforcing material. Each of the layers (2), (3) and (4) are typically in fibrous form.

The multilayer material (1) further comprises a physiologically acceptable acid coated onto the fibres making up the reinforcing material to form the third layer (4).

In use, the multilayer material (1) can be rapidly applied to a wound site by removing any packaging and bringing either layer (2) or (3) into direct contact with the wound exudate. Beneficially, time can be saved as there is no need to analyse which surface of the multilayer material is the wound contacting surface, i.e. the surface that initially comes into direct contact with the wound exudate, since both layers (2, 3) are suitable.

Once applied, the physiologically acceptable acid dissolves in the wound exudate and contacts the chitosan. The dissolved acid reacts with the chitosan and/or chitosan derivative to form the corresponding salt of chitosan or chitosan derivative. The resultant salt gels upon contact with wound exudate, effectively encapsulating the fluid.

Furthermore, the reinforcing layer (4) typically comprises a superabsorbent material capable of absorbing wound exudate. Advantageously, this speeds up the time it takes to stem the bleeding from the wound.

### Examples

### Reference Example 1:

First and second layers, each being a 50 gsm layer of chitosan and Tencel®, non-woven, and coated with lactic acid (Blend of chitosan:Tencel® 55:45) between which lies a third layer of polyacrylate superabsorbent fibre (260 gsm), being a 50:50 blend of polyacrylate fibres and viscose fibres.

The layers were needle punched together.

### Reference Example 2:

First and second layers, each being a 135 gsm layer of chitosan and Tencel®, non-woven, and coated with lactic acid (Blend of chitosan:Tencel® 55:45) between which lies a third layer of polyacrylate superabsorbent fibre (260 gsm), being a 50:50 blend of polyacrylate fibres and viscose fibres.

The layers were needle punched together.

### Reference Example 3:

First and second layers, each being a 50 gsm layer of chitosan, non-woven, coated with lactic acid, between which lies a third layer of polyacrylate superabsorbent fibre (260 gsm), being a 50:50 blend of polyacrylate fibres and viscose fibres.

The layers were needle punched together.

### Reference Example 4:

First and second layers, each being a 135 gsm layer of chitosan, non-woven, coated with lactic acid, between which lies a third layer of polyacrylate superabsorbent fibre (260 gsm), being a 50:50 blend of polyacrylate fibres and viscose fibres.

The layers were needle punched together.

### Reference Example 5:

First and second layers, each being a 135 gsm layer of chitosan, non-woven, coated with lactic acid, between which lies a third layer of polyacrylate superabsorbent fibre (120 gsm), being a 50:50 blend of polyacrylate fibres and viscose fibres.

The layers were needle punched together.

### Reference Example 6:

First and second layers, each being a 135 gsm layer of chitosan, non-woven, coated with lactic acid, between which lies a dry laid viscose polyamide layer (37 gsm).

The layers were needle punched together.

### Reference Example 7:

First and second layers, each being a 135 gsm layer of chitosan, non-woven, coated with lactic acid, between which lies a dry laid viscose polyamide layer (37 gsm).

The layers are heat bonded together using a polycalprolactone adhesive.

### Example 8:

First and second layers, each being a 70gsm layer of chitosan, non-woven, between which lies a 70gsm layer of viscose, non-woven, coated with lactic acid.

The layers were needle punched together.

### Reference Example 1 dressing:

**Table 1: Absorbance and fluid retention data of the dressing of Example 1**

| | | Weight (gsm) | Absorbance (g/g) | Absorbance (g/100cm2) | Retention (%) |
|---|---|---|---|---|---|
| Trilayer | Mean | 441.3 | 10.6 | 46.5 | 92.5 |
| | Std.dev | 53.3 | 0.5 | 3.5 | 1.0 |

The results displayed in Table 1 show that the trilayer dressing of reference Example 1 was capable of achieving high absorbance and a high fluid retention of 92.5%.

The absorbency and tensile strength of the dressings of references Examples 1-7 and Example 8 were tested with comparative examples of single layer dressings. The results are shown in Table 2.

For tensile strength, the results for references Examples 1-7 show a value of >50 as the strength of the dressing was too high for load cell when dry (>50N). The wet strength was measured as >15N/25cm.

| Dressing example | Absorbency (g/100cm2) | Absorption under compression (g/100cm2) | Dry Tensile (N/25mm) | Wet Tensile (N/25mm) |
|---|---|---|---|---|
| Reference Example 1 | 46.5 | 32.5 | >50* | 17.2 |
| Reference Example 3 | 44.5 | 28.6 | >50* | 16.3 |
| Reference Example 4 | 56.8 | 41.2 | >50* | 20.1 |
| Reference Example 5 | 48.3 | 36.4 | >50* | 16.9 |
| Reference Example 6 | 25.6 | 15.2 | >50* | 27.6 |
| Reference Example 7 | 20.1 | 12.4 | >50* | 28.3 |
| Example 8 | 23.5 | 16.1 | 18.80 | 5.67 |
| Alginate dressing single layer | 21.28 | 13.52 | 12.40 | 9.88 |
| Alginate dressing single layer | 17.39 | 10.28 | 0.21 | 0.52 |
| Carboxymethylcellulose dressing single layer | 19.68 | 15.96 | 10.59 | 0.60 |
| Carboxymethycellulose dressing single layer with reinforced stitch bonding | 27.93 | 18.8 | 37.19 | 15.69 |
| Gelling fibre cellulose dressing single layer | 23.75 | 18.08 | 13.63 | 1.23 |
| Chitosan gelling fibre dressing single layer | 28.59 | 16.12 | 23.69 | 2.16 |

| | | | | |
|---|---|---|---|---|
| * Reading greater than load cell capacity of tensiometer | | | | |

Table 2: Absorbance and tensile strength data for test dressing. The test methods are as follows:

### Absorbency:

A 5cm x 5cm area of dressing was prepared and weighed. The test article was then immersed in saline solution (de-ionised water with 0.9% sodium chloride) for a period of 30 minutes. Following immersion, the test article was removed and excess fluid was allowed to drain. The dressing was re-weighed and the weight of fluid absorbed per weight and area of test article was determined.

### Absorbency under compression:

A 5cm x 5cm area of dressing was prepared and weighed. The test article was applied to a test rig whereby a weight mimicking 40mmHg pressure was applied on top. Saline solution (de-ionised water with 0.9% sodium chloride) was introduced to the test article and allowed to stand for a period of 30 minutes. Following absorbency, the test article is removed and excess fluid was allowed to drain. The dressing was re-weighed and the weight of fluid absorbed per weight and area of test article was determined.

### Dry Tensile:

The test article was prepared to a width of 25mm and positioned within a tensiometer. The force to break the test article was recorded.

### Wet Tensile:

The test article was prepared to a width of 25mm. 2g of fluid (saline solution of de-ionised water with 0.9% sodium chloride) was applied to the central portion of the test article, allowing it to absorb for a period of 30 minutes. The test article was then positioned within a tensiometer. The force to break the test article was recorded.

The results displayed in Table 2 show that the wet tensile strength of the dressings of the present invention is increased at least compared to the Chitosan gelling fibre single layer.

It is of course to be understood that the present invention is not intended to be restricted to the foregoing examples which are described by way of example only.

## Claims

1. A multilayer material comprising first and second layers comprising a chitosan and/or a chitosan derivative and a third layer located between the first and second layers comprising a reinforcing material, wherein the chitosan derivative is selected from the group consisting of partially deacetylated chitin, carboxymethyl chitosan, hydroxyl butyl chitin, N-acyl chitosan, O-acyl chitosan, N-alkyl chitosan, O-alkyl chitosan, N-alkylidene chitosan, O-sulfonyl chitosan, sulfated chitosan, phosphorylated chitosan, nitrated chitosan, alkalichitin, alkalichitosan, or metal chelates with chitosan, wherein the third layer comprises a physiologically acceptable acid selected from the group consisting of formic acid, acetic acid, ascorbic acid, halogen acetic acids, propanoic acid, propenoic acid, lactic acid, succinic acid, acrylic acid, glyoxylic acid, pyruvic acid, hydroxyl propionic/butanoic acid, sulphuric acid, hydrochloric acid, and combinations of any two or more thereof.

2. A multilayer material according to claim 1, wherein the physiologically acceptable acid is also located in the first and/or second layer of the multilayer material.

3. A multilayer material according to claim 2, wherein the acid is coated onto the chitosan and/or the chitosan derivative.

4. A multilayer material according to claim 2, wherein the first and/or second layers further comprise a carrier material for the acid, optionally wherein the acid is absorbed in, or coated onto, the carrier material.

5. A multilayer material according to any preceding claim, wherein the reinforcing material comprises a non-absorbent material, for example viscose; or wherein the reinforcing material comprises an absorbent material.

6. A multilayer material according to claim 5, wherein the absorbent material is a superabsorbent material.

7. A multilayer material according to claim 6, wherein the superabsorbent material is a polymeric material, such as poly(vinyl alcohol) (PVA), poly(ethylene oxide) (PEO), and poly(acrylic acid).

8. A multilayer material according to any preceding claim, wherein the chitosan, chitosan derivative and/or the reinforcing material are in the form of fibres.

9. A multilayer material according to any preceding claim, wherein the chitosan derivative is a partially deacetylated chitin, optionally with a deacetylation of degree above about 50%.

10. A multilayer material according to any preceding claim, wherein the acid is coated onto the reinforcing material.

11. A wound dressing comprising a multilayer material according to any of claims 1 to 10.

12. A method of manufacturing a multilayer material comprising the steps of:
(a) providing first and second layers comprising chitosan and/or a chitosan derivative;
(b) attaching the first and second layers to a third layer comprising a reinforcing material, such that the third layer is located between the first and second layers,
wherein the third layer further comprises a physiologically acceptable acid selected from the group consisting of formic acid, acetic acid, ascorbic acid, halogen acetic acids, propanoic acid, propenoic acid, lactic acid, succinic acid, acrylic acid, glyoxylic acid, pyruvic acid, hydroxyl propionic/butanoic acid, sulphuric acid, hydrochloric acid, and combinations of any two or more thereof.

13. A method according to claim 12, comprising the steps of:
(a)
(i) mixing a chitosan and/or chitosan derivative with a non-aqueous solvent; and
(ii) mixing a reinforcing material or carrier material with a solvent;
(b) adding a physiologically acceptable acid selected from the group consisting of formic acid, acetic acid, ascorbic acid, halogen acetic acids, propanoic acid, propenoic acid, lactic acid, succinic acid, acrylic acid, glyoxylic acid, pyruvic acid, hydroxyl propionic/butanoic acid, sulphuric acid, hydrochloric acid, and combinations of any two or more thereof to the or each mixture;
(c) removing the solvent from the or each mixture to provide an acid coated component(s) being an acid coated chitosan, an acid coated chitosan derivative, a mixture of acid coated chitosan and chitosan derivative, an acid coated reinforcing material and/or an acid coated carrier material;
(d) attaching first and second layers comprising chitosan and/or a chitosan derivative to a third layer located between the first and second layers comprising a reinforcing material,
wherein one or more of the first and second layers comprises the acid coated chitosan, chitosan derivative and/or acid coated carrier material; and wherein the third layer comprises the acid coated reinforcing material.

14. A method according to claim 13, wherein the acid coated chitosan and/or chitosan derivative is attached to the reinforcing material by heat bonding or needle punching.

15. A multilayer material according to any of claims 1 to 10, or a wound dressing according to claim 11, for use in the treatment of a wound, in absorbing fluid discharged from a wound, or in stemming a flow of a fluid discharged from a wound.

## Patentansprüche

1. Mehrschichtmaterial, umfassend erste und zweite Schichten, die ein Chitosan und/oder ein Chitosanderivat umfassen, und eine dritte Schicht, die zwischen den ersten und zweiten Schichten lokalisiert ist, die ein Verstärkungsmaterial umfasst, wobei das Chitosanderivat ausgewählt ist aus der Gruppe bestehend aus deacetylisierten Chitin, Carboxymethylchitosan, Hydroxylbutylchitin, N-Acylchitosan, O-Acylchitosan, N-Alkylchitosan, O-Alkylchitosan, N-Alkylidenchitosan, O-Sulfonylchitosan, sulfatiertes Chitosan, phosphoryliertes Chitosan, nitriertes Chitosan, Alkalichitin, Alkalichitosan, oder Metallchelate mit Chitosan, wobei die dritte Schicht eine physiologisch verträgliche Säure umfasst, ausgewählt aus der Gruppe bestehend aus Ameisensäure, Essigsäure, Ascorbinsäure, Halogenessigsäuren, Propansäure, Propensäure, Milchsäure, Bernsteinsäure, Acrylsäure, Glyoxylsäure, Brenztraubensäure, Hydroxylpropion/Butansäure, Schwefelsäure, Salzsäure und Kombinationen von jeglichen zwei oder mehr davon.

2. Mehrschichtmaterial nach Anspruch 1, wobei die physiologisch verträgliche Säure ferner in der ersten und/oder der zweiten Schicht des Mehrschichtmaterials lokalisiert ist.

3. Mehrschichtmaterial nach Anspruch 2, wobei die Säure auf das Chitosan und/oder das Chitosanderivat beschichtet ist.

4. Mehrschichtmaterial nach Anspruch 2, wobei die erste und/oder die zweite Schicht ferner ein Trägermaterial für die Säure umfasst, wobei optional die Säure in dem Trägermaterial absorbiert oder darauf beschichtet ist.

5. Mehrschichtmaterial nach einem der vorhergehenden Ansprüche, wobei das Verstärkungsmaterial ein nicht absorbierendes Material umfasst, beispielsweise Viskose; oder wobei das Verstärkungsmaterial ein absorbierendes Material umfasst.

6. Mehrschichtmaterial nach Anspruch 5, wobei das absorbierende Material ein superabsorbierendes Material ist.

7. Mehrschichtmaterial nach Anspruch 6, wobei das superabsorbierende Material ein Polymermaterial ist, beispielsweise Poly(vinylalkohol) (PVA), Poly(ethylenoxid) (PEO), und Poly(acrylsäure).

8. Mehrschichtmaterial nach einem der vorhergehenden Ansprüche, wobei das Chitosan, das Chitosanderivat und/oder das Verstärkungsmaterial in der Form von Fasern ist.

9. Mehrschichtmaterial nach einem der vorhergehenden Ansprüche, wobei das Chitosanderivat ein teilweise deacetyliertes Chitin ist, optional mit einer Deacetylierung mit einem Grad oberhalb von 50%.

10. Mehrschichtmaterial nach einem der vorhergehenden Ansprüche, wobei die Säure auf das Verstärkungsmaterial beschichtet ist.

11. Wundverband, umfassend ein Mehrschichtmaterial nach einem der Ansprüche 1 bis 10.

12. Verfahren zur Herstellung eines Mehrschichtmaterials wie in Anspruch 1 definiert, umfassend die folgenden Schritte:
(a) Bereitstellen von ersten und zweiten Schichten, die Chitosan und/oder ein Chitosanderivat umfassen;
(b) Anbringen der ersten und zweiten Schichten an einer dritten Schicht, die ein Verstärkungsmaterial umfasst, derart, dass die dritte Schicht zwischen den ersten und zweiten Schichten lokalisiert ist.
wobei die dritte Schicht ferner eine physiologisch verträgliche Säure umfasst, ausgewählt aus der Gruppe bestehend aus Ameisensäure, Essigsäure, Ascorbinsäure, Halogenessigsäuren, Propansäure, Propensäure, Milchsäure, Bernsteinsäure, Acrylsäure, Glyoxylsäure, Brenztraubensäure, Hydroxylpropion/Butansäure, Schwefelsäure, Salzsäure und Kombinationen von jeglichen zwei oder mehr davon.

13. Verfahren nach Anspruch 12, umfassend die folgenden Schritte:
(a)
(i) Mischen eines Chitosans und/oder eines Chitosanderivats mit einem nicht wässrigen Lösungsmittel; und
(ii) Mischen eines Verstärkungsmaterial oder Trägermaterials mit einem Lösungsmittel;
(b) Hinzufügen einer physiologisch verträglichen Säure, ausgewählt aus der Gruppe bestehend aus Ameisensäure, Essigsäure, Ascorbinsäure, Halogenessigsäuren, Propansäure, Propensäure, Milchsäure, Bernsteinsäure, Acrylsäure, Glyoxylsäure, Brenztraubensäure, Hydroxylpropion/Butansäure, Schwefelsäure, Salzsäure und Kombinationen von jeglichen zwei oder mehr davon zu der oder jeder Mischung;
(c) Entfernen des Lösungsmittels von der oder jeder Mischung, um eine säurebeschichtete Komponente(n) bereitzustellen, die ein säurebeschichtetes Chitosan, ein säurebeschichtetes Chitosanderivat, eine Mischung von säurebeschichtetem Chitosan und Chitosanderivat, ein säurebeschichtetes Verstärkungsmaterial und/oder ein säurebeschichtetes Trägermaterial ist;
(d) Anbringen erster und zweiter Schichten, die Chitosan und/oder ein Chitosanderivat umfassen, an einer dritten Schicht, die zwischen den ersten und zweiten Schichten lokalisiert ist, umfassend ein Verstärkungsmaterial,
wobei eine oder mehr von den ersten und zweiten Schichten das säurebeschichtete Chitosan, Chitosanderivat und/oder säurebeschichtete Trägermaterial umfasst; und wobei die dritte Schicht das säurebeschichtete Verstärkungsmaterial umfasst.

14. Verfahren nach Anspruch 13, wobei das säurebeschichtete Chitosan und/oder Chitosanderivat an dem Verstärkungsmaterial mittels einer Hitzeverbindung oder einer Vernadelung angebracht wird.

15. Mehrschichtmaterial nach einem der Ansprüche 1 bis 10, oder Wundverband nach Anspruch 11, zur Verwendung bei der Behandlung einer Wunde, beim Absorbieren eines Fluids, das von einer Wunde abgegeben wird, oder beim Aufhalten eines Flusses eines Fluids, das von einer Wunde abgegeben wird.

## Revendications

1. Matière stratifiée comprenant des première et deuxième couches, comprenant un chitosane et/ou un dérivé de chitosane, et une troisième couche disposée entre les première et deuxième couches et comprenant une matière de renfort, dans laquelle le dérivé de chitosane est choisi dans le groupe consistant en la chitine désacétylée partiellement, le carboxy méthyl chitosane, l'hydroxyl butyl chitine, un N-alcoylchitosane, un O-alcoyl chitosane, un N-alcoylidène chitosane, l'O-sulfonyl chitosane, le chitosane sulfaté, le chitosane phosphorylé, le chitosane nitré, une alcalichitine, un alcalichitosane ou des chélates métalliques avec du chitosane, dans laquelle la troisième couche comprend un acide acceptable physiologiquement, choisi dans le groupe consistant en l'acide formique, l'acide acétique, l'acide ascorbique, les acides haloacétiques, l'acide propanoïque, l'acide propènoïque, l'acide lactique, l'acide succinique, l'acide acrylique, l'acide glyoxylique, l'acide pyruvique, l'acide hydroxyl-propionique/ butanoïque, l'acide sulfurique, l'acide chlorhydrique et des combinaisons de deux ou plusieurs d'entre eux.

2. Matière stratifiée suivant la revendication 1, dans laquelle l'acide acceptable physiologiquement est disposé aussi dans la première et/ou la deuxième couche de la matière stratifiée.

3. Matière stratifiée suivant la revendication 2, dans laquelle l'acide est appliqué en revêtement au chitosane et/ou au dérivé de chitosane.

4. Matière stratifiée suivant la revendication 2, dans laquelle la première et/ou la deuxième couche comprend en outre une matière de support de l'acide, dans laquelle éventuellement l'acide est absorbé dans la matière de support ou y est appliqué en revêtement.

5. Matière stratifiée suivant l'une quelconque des revendications précédentes, dans laquelle la matière de renfort comprend une matière non absorbante, par exemple de la viscose ; ou dans laquelle la matière de renfort comprend une matière absorbante.

6. Matière stratifiée suivant la revendication 5, dans laquelle la matière absorbante est une matière super-absorbante.

7. Matière stratifiée suivant la revendication 6, dans laquelle la matière super-absorbante est une matière polymère, telle que de l'alcool polyvinylique (PVA), du poly (oxyde d'éthylène) (PO), et du poly (acide acrylique).

8. Matière stratifiée suivant l'une quelconque des revendications précédentes, dans laquelle le chitosane, le dérivé de chitosane et/ou la matière de renfort sont sous la forme de fibres.

9. Matière stratifiée suivant l'une quelconque des revendications précédentes, dans laquelle le dérivé de chitosane est une chitine désacétylée partiellement, éventuellement ayant une désacétylation d'un degré supérieur à environ 50%.

10. Matière stratifiée suivant l'une quelconque des revendications précédentes, dans laquelle l'acide est appliqué en revêtement à la matière de renfort.

11. Pansement de plaie comprenant une matière stratifiée suivant l'une quelconque des revendications 1 à 10.

12. Procédé de fabrication d'une matière stratifiée telle que définie à la revendication 1, comprenant les stades dans lesquels :
a) On se procure des première et deuxième couches comprenant du chitosane et/ou un dérivé de chitosane ;
b) on adjoint les première et deuxième couches à une troisième couche comprenant une matière de renfort de manière à ce que la troisième couche soit placée entre les première et deuxième couches ;
dans lequel la troisième couche comprend en outre un acide acceptable physiologiquement, choisi dans le groupe consistant en l'acide formique, l'acide acétique, l'acide ascorbique, les acides haloacétiques, l'acide propanoïque, l'acide propènoïque, l'acide lactique, l'acide succinique, l'acide acrylique, l'acide glyoxylique, l'acide pyruvique, l'acide hydroxyl-propionique/ butanoïque, l'acide sulfurique, l'acide chlorhydrique et des combinaisons de deux ou plusieurs d'entre eux.

13. Procédé suivant la revendication 12, comprenant les stades dans lesquels :
a)
(i) On mélange un chitosane et/ou un dérivé de chitosane à un solvant non aqueux et
(ii) on mélange une matière de renfort ou une matière de support à un solvant ;
b) on ajoute un sel acceptable physiologiquement choisi dans le groupe consistant en l'acide formique, l'acide acétique, l'acide ascorbique, les acides haloacétiques, l'acide propanoïque, l'acide propènoïque, l'acide lactique, l'acide succinique, l'acide acrylique, l'acide glyoxylique, l'acide pyruvique, l'acide hydroxyl-propionique/ butanoïque, l'acide sulfurique, l'acide chlorhydrique et des combinaisons de deux ou plusieurs d'entre eux à le ou à chaque mélange ;
c) on élimine le solvant du ou de chaque mélange pour obtenir un constituant ou des constituants revêtus d'acide, qui sont un chitosane revêtu d'acide, un dérivé de chitosane revêtu d'acide, un mélange de chitosane et de dérivé de chitosane revêtu d'acide et une matière de renfort revêtue d'acide et/ou une matière de support revêtue d'acide ;
d) on adjoint des première et deuxième couches comprenant du chitosane et/ou un dérivé de chitosane à une troisième couche disposée entre les première et deuxième couches comprenant une matière de renfort,
dans lequel une ou plusieurs des première et deuxième couches comprend le chitosane, un dérivé de chitosane revêtu d'acide et/ou la matière de support revêtue d'acide ; et dans lequel la troisième couche comprend la matière de renfort revêtue d'acide.

14. Procédé suivant la revendication 13 dans lequel le chitosane et/ou un dérivé de chitosane revêtu d'acide est adjoint à la matière de renfort par liaison par la chaleur ou par aiguilletage.

15. Matière stratifiée suivant l'une quelconque des revendications 1 à 10, ou pansement de plaie suivant la revendication 11, à utiliser dans le traitement d'une plaie, dans du fluide absorbant évacué d'une plaie ou dans l'endiguement d'un courant d'un fluide évacué d'une plaie.
